# EUROPEAN PATENT SPECIFICATION

(11) **EP 3 985 370 B1**
(45) Date of publication and mention of the grant of the patent: **28.05.2025**
(21) Application number: 20823215.7
(22) Date of filing: 15.06.2020
(51) Int. Cl.: A61B 17/88, A61C 8/00, A61C 19/04, B25B 23/142, A61B 90/00, A61B 5/00

(54) **ELECTRONIC TORQUE METER DESIGNED TO MEASURE AND SEND READING DATA OVER A WIRELESS INTERFACE AND SYSTEM INCLUDING SAID TORQUE METER**
ELEKTRONISCHER DREHMOMENTMESSER ZUM MESSEN UND SENDEN VON LESEDATEN ÜBER EINE DRAHTLOSE SCHNITTSTELLE UND SYSTEM MIT DIESEM DREHMOMENTMESSER
COUPLEMÈTRE ÉLECTRONIQUE APTE À MESURER ET ENVOYER DES DONNÉES DE LECTURE VIA UNE INTERFACE SANS FIL ET SYSTÈME COMPRENANT LEDIT COUPLEMÈTRE

(30) Priority: 13.06.2019 BR 102019012058
(43) Date of publication of application: 20.04.2022
(73) Proprietor: M3 Health Indústria e Comércio de Produtos Médicos, Odontológicos e Correlatos S.A., CEP: 13212-213 Jundiaí (BR)
(72) Inventor: BLAY, Alberto, São Paulo, SP CEP 01250-040 (BR); MESSADDEQ, Younès, Quebec, Quebec (CA); BHARUCHA, Eric, G3A 2A1, St- Augustin Quebec (CA); NUCINI, Alex Araújo, Santa Rosa de Viterbo CEP 14270-000 (BR); TEGON, André de Palma, Itatiba, SP CEP 13251-500 (BR); MATSUBARA, Elaine Yoshiko, São Paulo, SP CEP 01307-012 (BR); SPECIAN, Sybele Saska, Tamboré, Santana de Parnaíba, SP CEP 06543-003 (BR)
(74) Representative: Pereira Garcia, João Luís
(86) International application number: PCT/BR2020/050210
(87) International publication number: WO 2020/248037

(56) References cited:
- EP-A1- 2 221 022
- EP-B1- 2 922 485
- WO-A1-2013/183069
- WO-A1-2014/082087
- BR-A2- PI1 102 343
- BR-B1- 102018 010 649
- US-A1- 2011 004 199
- US-A1- 2012 006 161
- US-A1- 2015 351 819
- US-B2- 8 443 703

## Description

### INVENTION FIELD

. The present invention belongs to Dentistry and Medicine Fields, particularly related to implants, rigid internal fixation systems and other medical devices, more precisely to implants and dental or orthopedic prostheses.

. The present invention relates to a torque wrench as defined in the appended set of claims.

The present invention then describes a dental or medical torque wrench being digital electronic for implant or prosthesis placement surgeries being dental or medical. Its objective is to accurately present torque data, in real time, in order to generate a sequence of archived data for better analysis by experts and also to avoid or reduce the incidence of implant and prosthesis losses caused by incorrect torque application. The present invention also refers to a system comprising said torque wrench and a console for recording, processing and analyzing the data obtained.

### FUNDAMENTALS OF THE INVENTION

. The precision of the torque applied in the final installation procedure of dental implants or prosthetic components is a critical surgical step, which requires sensitivity and dexterity from the surgeon. The use of contra-angles as an instrument for installation is well known in the field of implantology, as they have rotating devices coupled to motors, which allow mechanical precision in the applied torque. However, manual installation using keys or ratchets also has its supporters and professionals who defend its advantages.

. In the current market, only contra-angles with speed reduction allow insertion of the implant/component with a torque free from mechanical failure. Although motors generally provide an accurate reading of the torque transmitted to the implant, if the contra-angle is not able to reduce speed fast enough, the torque marked on the motor will not be effectively communicated.

. Existing instruments for manual installation, on the other hand, allow all steps to be manually controlled, according to the user's discretion. However, when relying entirely on the surgeon's visual measurement, inexact torques can become recurrent, even when the instrument is used by qualified and experienced professionals.

. Also, currently, two types of torque wrench are available on the market for the purposes described here: surgical torque wrench and prosthetic torque wrench.

. As the name implies, the surgical torque wrench is indicated for use during the process of inserting the medical device into the surgical site. Its purpose is to guarantee the precision of the torque applied in the surgery in order to preserve the prosthesis or implant and ensure its ideal placement.

. On the other hand, the prosthetic torque wrench is indicated for fixing the temporary or definitive prosthesis on the implant.

. It is more common to find in the market mechanical torque wrenches that present less precision in their measurement, besides not allowing the archiving of generated/measured data.

. We highlight below some teachings of the state of the art that refer to this subject:

. The patent document BR 11 2015 012031 8 describes a torque wrench (torque wrench) for use in driving fasteners. The switch includes an electronic unit disposed in a housing fitted with the switch body that is capable of detecting and measuring the torque applied to a fastener by the switch and providing a torque level output to the user. The data is stored in the device, not being sent to another device via the wireless interface. It is not suitable for the purposes presented here.

**.** On the other hand, the document BR 10 2018 010649 0 describes a digital electronic dental torque wrench for dental implant placement surgeries and/or placement of prostheses on a dental implant, with components that allow the reduction of the incidence of loss of implants and prosthetic components that are caused by the application of inadequate torque and that the record in electronic files is made, containing the torque values applied during its use, increasing the predictability and traceability of surgical and prosthetic procedures in the field of implant dentistry. It is a digital electronic torque wrench that stores the digital data obtained from the measurements. However, the data is stored in the device and is not sent to another device via the wireless interface. In addition, the electronic circuit must be removed to be sterilized by autoclaving.

**.** The document BR 0114546-0 describes an apparatus, a system and a method for providing a dental diagnosis and/or a dental utensil to a user by examining a portion of a user's mouth. The user can also take images of their mouth by placing a ruler or other measuring device adjacent to their mouth. Images can then be transmitted to an individual and/or a central processing unit for diagnosis. However, it does not apply for torque measurement.

**.** The document US 9,168,418 describes a portable physical therapy/rehabilitation/exercise device to assess a patient's condition and to assess and track the progress made by a patient over time. During exercises in each mode, all data regarding forces (compression, tension, torque and the like) are stored in the device along with other types of quantitative and qualitative data. The stored data can be uploaded to the internet or external computer system to be analyzed later. It does not specifically deal with torque measurement for dental application.

**.** On the other hand, the document DE 19820640 describes a device that indicates a maximum allowed load that is entered into a computer. A partial load value is determined from an actual treatment and added to a condition-specific total load value obtained from previous treatments. The maximum allowable load is compared to the updated total load. A change signal is issued if the entire amount of charge reaches or exceeds the allowable charge. The amount of load is determined from the count of revolutions, torque and duration of dental treatment.

**.** The document WO 03/092526 deals with a medical treatment device comprising a pedal-operated control unit and a handpiece designed for a medical instrument, said handpiece being adapted to be controlled through said actuated control unit per pedal. Said part comprises monitoring devices to control data to operate the medical instrument, said data can be processed in an information system, installed in the control unit operated by pedals and data exchange with said handpiece. Thus, it is a device that stores data and treats them digitally in the device itself.

**.** The document CN 204698716 describes a manual dental device including a button adjustment and cell phone host computer, the transfer line end of this button adjustment device connect to the mobile main part, this button adjustment device is equipped with mechanism of power limit to exceed by presetting the limit value on the button. If the value reaches the maximum value, there is a break in the torque transmission between the button adjustment device and the main part of the cell phone.

**.** On the other hand, the document KR 20110074949 refers to a measuring device for the stability of the integrated dental implant to prevent a part of the screw of an implant from being damaged and to obtain an accurate data. In this apparatus, a torque wrench is used to connect a connecting piece of an internal screw and the transducer. A moving film camera confirms the location of a screen in a control box. A portion of the percussion pole body easily approaches the transducer using a distance sensor and an angle sensor. A percussion bar hits the transducer body with constant power.

. The document BR 1102343 refers to a digital dental torque wrench, which integrates to the construction of the mechanical device an electronic system that makes it possible to measure the torque force dimensioned in the project, allowing the maximum tightening, without risk of damaging the material, in addition to predicting the possibility of data storage during surgery. There is no mention of sending data over a wireless interface.

. The document EP 2 055 434 describes a screwdriver made of an antibiotic material. The screwdriver includes a nose blade corresponding to screw heads of various shapes and a rope-shaped shaft made of metallic wire or resin wire with sufficient flexibility, softness and recovery properties. A rotation gauge or digital torque display device is disposed at an open end of the shaft. It is described that this key further comprises a display comprising a tightening torque sensor disposed on the handle body and a shaft comprising a signal conductor consisting of the string-shaped shaft or a string-shaped portion for transferring signals from the sensor tightening torque, wherein the torque display portion has a circular shape concentric with the rope-shaped shaft and the rotational axis of the handle body, or the torque display portion has a display device disposed on a housing concentrically with the rope-shaped shaft and the rotational shaft of the handle body, and the tightening torque is indicated by light, sound or digital display in a small cross section on one side of the opposite box next to the part of the screen with connected torque to the axis.

**.** The document US 8,021,151 describes a surgical device that includes a manual instrument equipped with a rotary motor for driving a tool and at least one sensor for determining at least one operating parameter of the motor. The device also includes an electronic engine servo-control module and a flexible connection to electrically connect the instrument to the electronics module. The instrument includes a digital electronic circuit to process the sensor output signals and transmit them to the electronics module through the flexible connection.

**.** And the document US 7,002,469 describes a remote display unit for displaying remotely generated data without requiring a doctor to turn his head away from the doctor's object of attention. It is a method for communicating dental data to a user comprising providing a remote display unit including a housing connected to a lock and a display for communicating dental data to the user such that the user simultaneously views the dental data and an object of a dental procedure; and holding the housing close to the object within the user's view to communicate the dental data to the user who still receives on the remote display unit a control signal carrying the dental data.

WO 2014/082087 A1 discloses a medical electronic torque wrench configured to measure the torque exerted and to wirelessly communicate said measurement to an external control unit, the wrench forming an enclosure that enables the wrench to be autoclaved for sterilization purposes without damaging the battery assembly, the electronics unit or the sensors enclosed therein.

**.** Therefore, there is no solution in the state of the art equivalent to the one presented here in the present invention, which combines technical differentials, practicality, economic advantages, safety and reliability.

### OBJECTIVES OF THE INVENTION

**.** Aiming to supply the lag of torque instruments with high precision in the market, the present invention aims to develop an electronic torque wrench with signal emission via wireless technology, which allows active measurement of torque in real time during insertion manual of implants/screws or products from other related medical fields.

**.** Thus, it is an object of the present invention to provide a digital electronic torque wrench that sends data to an external device via a wireless interface.

**.** It is another of the objectives of the present invention to provide a digital electronic torque wrench that can be used for prosthetic implants and other medical devices that require application of torque application for their proper performance and purpose for their intended use.

**.** It is another objective of the present invention to provide a digital electronic torque wrench that comprises miniaturized components so that the device has the ideal size for use by users in addition to specific embedded software.

**.** It is another objective of the present invention to provide a digital electronic torque wrench that provides torque measurement with high accuracy.

**.** It is another objective of the present invention to provide a digital electronic torque wrench that can be sterilized in a standard autoclave process without the need to disassemble and remove its electronic components.

**.** It is yet another objective of the invention to provide a device for clinical applications including dental, maxillofacial and orthopedic surgery, in addition to prosthetic procedures.

**.** It is further an objective of the present invention to provide a digital electronic torque wrench that generates and sends data sequence that can be analyzed and processed in external devices such as computer, laptop, cell phone, wearable devices and other electronic devices with wireless interface.

### SUMMARY OF THE INVENTION

**.** The present invention achieves these and other objectives through an electronic torque wrench particularly used in dental and/or medical devices which comprises:
- an upper portion comprising a head socket and a ratchet mechanism;
- a lower portion;
- a central portion comprising sensors, an RF antenna, an RF transmitter, a circuit board, a microprocessor circuit, all these electronic components being resistant to high temperatures and sealed in said central portion which further comprises a window covering the entire said portion being welded,
where the torque data is read and sent to an external console for recording, treatment and analysis.

**.** The present invention achieves these and other objectives by means of an electronic torque wrench particularly used in dental and/or medical devices that comprises a central portion comprising a window welded onto an RF transmitter (8) and an antenna (3), the window being made of material that allows the passage of RF radiation.

**.** Furthermore, the present invention achieves these and other objectives by means of an electronic torque wrench particularly used in dental and/or medical devices which comprises a central portion comprising electronic components being sensors, an RF antenna, an RF transmitter, a circuit board, a microprocessor circuit, all being high temperature resistant and sealed in said central portion which further comprises a window covering the entire said portion being welded.

**.** Furthermore, the present invention achieves these and other objectives through an electronic torque wrench particularly used in dental and/or medical devices which comprises:
- a central portion comprising electronic components resistant to high temperatures and sealed in said central portion which further comprises a window covering the entire said portion being welded,

Being subjected to temperatures and pressures associated with sterilization procedures such as autoclave processes, disassembling the central portion for such procedures.

**.** Furthermore, the present invention achieves these and other objectives through a system particularly used in dental and/or medical procedures which comprises:
- a torque wrench as defined above;
- a console with embedded software;
- a ratchet fixed to said torque wrench;

All data obtained through the use of the torque wrench are sent to the console for recording, treatment and analysis.

### BRIEF DESCRIPTION OF THE DRAWINGS

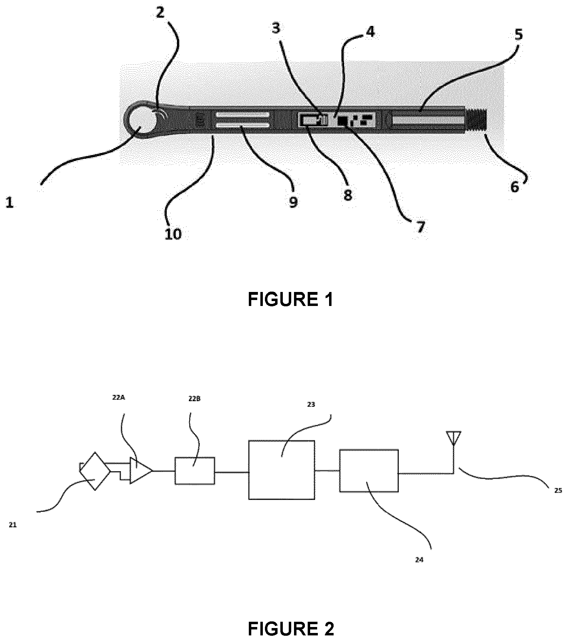

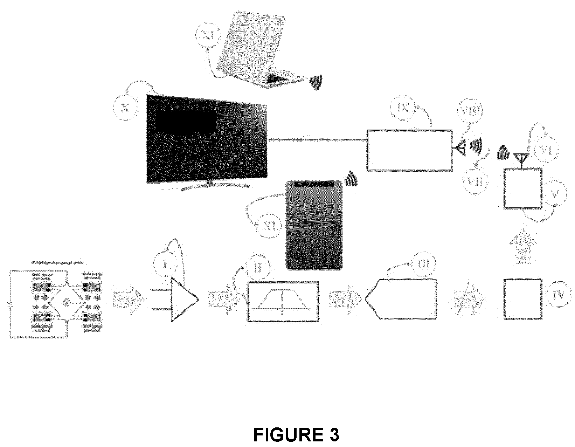

**.** The present invention will be described based on the drawings attached herein, which illustrate:
**.** Figure 1 illustrates the physical layout of a preferred embodiment of the torque wrench object of the present invention;
**.** Figure 2 illustrates the architecture of the torque wrench object of the present invention;
**.** Figure 3 illustrates the flow of processing the electronic data obtained through the torque wrench of the present invention.

### DETAILED DESCRIPTION OF THE INVENTION

**.** The present invention refers to an electronic torque wrench particularly used in dental and/or medical devices which comprises:
- an upper portion comprising a head socket and a ratchet mechanism;
- a lower portion;
- a central portion comprising sensors, an RF antenna, an RF transmitter, a circuit board, a microprocessor circuit, all these electronic components being resistant to high temperatures and sealed in said central portion which also comprises a window that covers the entire said portion being welded, the torque data being read and sent to an external console for recording, processing and analysis. The present invention further relates to an electronic torque wrench particularly used in dental and/or medical devices which comprises a central portion comprising a window welded onto an RF transmitter and an antenna, the window being made of material that allows the passage of RF radiation.

**.** The present invention further relates to an electronic torque wrench particularly used in dental and/or medical devices comprising a central portion comprising electronic components being sensors, an RF antenna, an RF transmitter, a circuit board, a microprocessor circuit, being all resistant to high temperatures and sealed in said central portion which further comprises a window covering the entire said portion being welded.

**.** The present invention further relates to an electronic torque wrench particularly used in dental and/or medical devices which comprises:
- a central portion comprising electronic components resistant to high temperatures and sealed in said central portion which further comprises a window that covers the entire said portion being welded, being subjected to temperatures and pressures associated with sterilization procedures such as autoclave processes without disassembly the central portion for such procedures.

**.** The present invention further relates to a system particularly used in dental and/or medical procedures which comprises:
- a torque wrench as described herein;
- a console with embedded software;
- a ratchet fixed to said torque wrench;

All data obtained through the use of the torque wrench are sent to the console for recording, treatment and analysis.

**.** The electronic torque wrench of the present invention was developed to be particularly used in dental and/or medical devices such as implants, prostheses and rigid fixation systems. It is noteworthy that said torque wrench transmits the torque applied by an operator to the ratchet 2 that will promote the fixation or placement of the implant or tightening of the prosthesis screw.

**.** The torque wrench object of the present invention is preferably indicated for:
- Implantology, periodontics, cranio-maxillofacial: installation of dental implants, prosthetic components and system of plates and screws for internal fixation;
- Orthopedics: installation of internal fixation plates and screws system, in addition to orthopedic prostheses in general.

**.** The present invention relates to a digital electronic torque wrench comprising three portions: an upper portion, a central portion and a lower portion. These portions are sequential and constitute the torque wrench object of the present invention, which has a preferably substantially cylindrical shape and its longitudinal axis is much larger than the diameter of its cross section. These portions are sequentially connected and surrounded by a metallic or polymeric body 10 that promotes the external finishing of said torque wrench.

**.** In a preferred embodiment of the present invention, the torque wrench has the following dimensions: 114 mm x 10 mm x 7 mm. These data show how its size is ideal to be used by an operator such as a surgeon. These proportions were reached especially due to the miniaturization of the electronic components present in this device.

**.** It is noteworthy that the torque wrench object of the present invention is made of material resistant to sterilization by moist heat such as autoclave or autoclaving.

**.** Preferably, the torque wrench of the present invention is made of titanium and its alloys or other medical grade metals such as medical grade stainless steel and further comprises a window (or viewfinder) located in its central portion made of a material that allows radiation of the RF pass through. This window allows the integration of a wireless transmission system.

**.** The digital electronic torque wrench or dental/medical torque wrench of the present invention is a device that allows the active real-time measurement of torque as a surgeon fixes dental/medical implants and screws. Torque readings are designed for a custom interface, preferably being mobile devices like smartphones or tablets.

**.** In the torque wrench of the present invention, miniaturized internal sensors will detect the torque applied to the upper portion of the torque wrench. The signal derived from these sensors is amplified and fed into a microcontroller based on the data acquisition system. The acquired data is then projected onto custom interface devices, such as smartphones or tablets, as long as they are within the signal range. Reading the generated data provides immediate feedback to the operator, allowing him to monitor the driving process and even stop at a pre-set level if desired. The system also has the advantage of being able to be sterilized by moist heat, ensuring safe reuse. The device can also be adapted for surgical procedures in other related medical fields.

**.** Data readings provide data to the operator allowing him to monitor the driving process and stop at a predefined level if desired. The system is no larger than existing mechanical equivalents and can be sterilized in a standard autoclave process. Inside the torque wrench of the present invention there are miniaturized sensors that detect the torque applied to the head of said torque wrench.

**.** The signal derived from these sensors is amplified and fed into a microcontroller based on the data acquisition system. The acquired data is transmitted wirelessly to nearby monitoring devices.

**.** As can be seen from Figure 1, in its upper portion there is a head socket 1 connected to a ratchet mechanism 2.

**.** In the lower portion of the torque wrench object of the present invention, opposite to the upper portion, is located the battery compartment 5 and a cover 6 located at the lower end of said torque wrench to access the compartment 5 in order to change the battery. The cover 6 can be screw-on or interference fit.

**.** In the central portion of the torque wrench of the present invention, there are sensor cavities 9 and an RF transmitter 8 which is a small electronic device used to transmit and/or receive signals in addition to the RF antenna 3 which is an electrical device that converts radio frequency into current alternating. The RF antenna 3 radiates the radio frequency energy generated in the transmitter 8 and fed to the antenna by a transmission line. And it directs energy in the desired directions in addition to suppressing radiation in unwanted directions. Thus, the reading of the torque measurement performed by sensors located in cavities 9 are transmitted by transmitter 8 by antenna 3.

**.** In a preferred embodiment, the sensors 9 form a bridge configuration that compensates for thermal drift.

**.** Further, in the central portion, there is a printed circuit board 4 and microprocessor circuits 7 that will work and convert the read data to be sent to preferably mobile devices. One of the advantages perceived in the present invention is that it does not need to disassemble the device to carry out the sterilization process by autoclaving.

**.** This fact is possible because the electronic components are sealed within a compartment and all of them are selected to be resistant to high temperatures. Also, electronic components are not exposed to moisture from the autoclave chamber. Medical grade epoxy materials and encapsulants can withstand the temperatures used in the autoclaving process. Laser welding is also used to seal the sensor components.

**.** It is noteworthy that such techniques are inspired by aerospace and automotive applications that use similar components that can withstand exposure to high temperature and humidity. Also, in preferred embodiments, special batteries that can withstand these temperatures are used.

**.** However, in a preferred embodiment, the torque wrench object of the present invention comprises an electronic circuit designed so as to be able to undergo repeated exposure to an autoclave sterilization environment.

**.** In the present invention, the electronic components are matrix semiconductors (die semiconductors), which means that the silicon is mounted directly on the printed circuit board (PCB) 4. Resistors and passive capacitors (not shown) are used, which are the smallest components. available. PCB 4 board is manufactured using state-of-the-art lithographic techniques currently available in Switzerland and Germany and are miniaturized via laser drilling. Blind and buried pathways techniques that provide the highest level of PCB integration are also used. Thus, it is possible to provide state-of-the-art electronic components that make the torque wrench of the present invention portable, light, practical, reproducible and with a high level of reliability during the entire manufacturing process.

**.** Further, in a preferred embodiment of the present invention, the torque wrench of the present invention comprises a colored light emitting diode or display panel that informs on how to directly read connection status information and a sound transducer that gives feedback and/or information status.

**.** Furthermore, a special adhesive mechanism is used to attach a transparent window to the central portion.

**.** In a preferred embodiment, the torque wrench of the present invention further comprises a memory preferably inserted in its central portion. This memory is used to record and store the data measured by the apparatus of the present invention.

**.** Further, said apparatus may comprise a multi-colored LED or display panel which presents reading information including connection status and a sound transducer which provides connection or reading information.

**.** Figure 2 illustrates the operation of the electronic components present in the device object of the present invention. As can be seen from the aforementioned figure, in the central portion of the torque wrench of the present invention, there is a thermally compensated bridge sensor 21 that receives the signal related to the torque measurement which is connected to an amplifier 22A which in turn is connected to filter and signal conditioning circuits 22B connected to a microprocessor 23 which takes the signal to an RF transmitter 24 forwarding the data to an antenna 25. Thus, the signal is sent via a wireless interface to an installed application or software on an internet device such as computer, laptop, tablet, smartphone, wearable device etc.

### Operation and performance of the torque wrench of the present invention

. The torque wrench of the present invention is preferably used according to the following steps:
a) When starting a surgical procedure that requires a medical device with torque application, based on an assessment of bone health and density, an operator (surgeon) will enter defined torque values and maximum torque values to be applied to the bone structure of the patient;
b) The surgeon will calibrate the device;
c) During surgery, the key is collected and a communication link is automatically established between the key and the host device;
d) The implant is screwed into place and the torque wrench provides live torque data to the surgeon;
e) When targets and limits are reached, indicators and alarms are triggered;
f) Data is instantly and automatically sent via wireless interface to an application installed on a device such as a computer, laptop, smartphone, tablets, among others, and is then recorded, which can be analyzed, treated, built a patient or treatment history, among others;
g) After surgery, the battery cover 6 and battery 5 devices are removed.

The key and cover 6 are sent for sterilization.

. In a preferred embodiment of the present invention, the torque wrench is capable of measuring torque ranging between 0 and 100 N.cm with decimal precision (0.1 N.cm).

### Operation of the software embedded in the console for processing the data read by the torque wrench of the present invention

. After linking the console to the torque wrench of the present invention using a code in said torque wrench, the data is transmitted to the gateway and the operation starts. If the link between the torque wrench and the gateway is lost, an alarm goes off. A color indicator present on the torque wrench constantly indicates link status information and other tips that can be programmed into the device. In a preferred embodiment of the present invention, a low battery, for example, is displayed as a flashing red light.

. Data is encrypted at the torque wrench and later decrypted at the gateway. It can then be uploaded to multiple display platforms. In yet another modality, the gateway can be a tablet device.

### Obtaining and processing data from readings

. The data read by the sensors present in the torque wrench of the present invention are treated according to the following steps as illustrated in Figure 3:
(I) Data from the sensors is amplified;
(II) Data passes through a filter;
(III) The signal is then digitized in an analog to digital converter;
(IV) The signal is scaled corrected for calibration and encrypted in the microprocessor;
(V) Signal flow is fed into a BLE module;
(VI) The signal is transmitted via antenna;
(VII) Signal passes through air waves;
(VIII) Signal is received by gateway antenna;
(IX) The signal is fed into the gateway;
(X) The signal is interpreted in graphical formats for display on a monitor;

**.** In a preferred embodiment, data is obtained and processed following the steps below:
- sensor data is in the form of electrical voltage changes. These changes are amplified by an instrumentation amplifier;
- the data is then filtered using a 4th order bandpass filter of the low voltage front filter, which removes noise before entering an ADC or analog to digital converter;
- once digitized, the data points are scaled and an algorithm is used that maps voltage to torque using a calibration table;
- essentially, the more torque applied, the greater the voltage difference measured between the sensors;
- once converted into torque, the data is sent to a low power Bluetooth module through a serial link between the microcontroller and the BLE module;
- the radio frequency link then transmits the data to a gateway module composed of a BLE receiving module and a small computer;
- this gateway redistributes data to monitors or be displayed as such on a gateway equipped with a display;
- In preferred embodiments a tablet can be used;
- Data is presented in GUI (general user interface) to the attending physician or dentist in the form of torque data or a variant presentation (a color scheme, for example).

**.** The present invention has numerous technical and economic advantages when compared to the state of the art, some of which are listed below:
- it is a digital electronic torque wrench capable of performing accurate torque measurements in dental and medical implant procedures, avoiding or reducing losses of implants and prosthetic components caused by the application of incorrect torque;
- is capable of sending the data collected through a wireless interface to mobile devices, preferably, such as smartphones and tablets, so that such data can be processed and analyzed in software and/or applications for this purpose. Thus, the recorded data generate a very useful history for the operator;
- it is a device that measures torque and transmits the said measurement via a wireless interface to a console or a device such as a smartphone or tablet for precise and quantitative manual adjustments;
- comprises miniaturized electronic circuits resistant to extreme environments such as: shockproof, extreme temperature proof, waterproof, among others;
- it is a light device, ideally sized and easy to use;
- the torque wrench of the present invention has a low production cost, easy assembly and operation. Battery consumption is still low.

**.** Having described an example of a preferred embodiment of the present invention, it is to be understood that the scope of the present invention encompasses other possible variations of the described inventive concept, being limited solely by the content of the appended claims, including possible equivalents therein.

## Claims

1. Electronic torque wrench configured to be used in dental and/or medical devices, said electronic torque wrench comprising:
- an upper portion comprising a head socket (1) and a ratchet mechanism (2);
- a lower portion;
- a central portion comprising sensors (9), an RF antenna (3), an RF transmitter (8), a circuit board (4), a microprocessor circuit (7), all these electronic components being resistant to temperatures associated with sterilization procedures such as autoclave processes and sealed in said central portion which further comprises a welded window covering the RF transmitter (8) and the antenna (3), the window being made of material that allows passing RF radiation; wherein the upper, central and lower portions are connected sequentially and wrapped in a metallic or polymeric body, wherein said electronic torque wrench comprises electronic components configured to be subjected to temperatures and pressures associated with sterilization procedures such as autoclave processes without disassembling the central portion for such procedures, wherein said sensors (9) are configured to detect a torque value applied to the upper portion and to generate a corresponding signal, said microprocessor circuit (7) is configured to encrypt and feed said signal to the RF transmitter (8), and said RF transmitter (8) is configured to send said encrypted signal to an external console through the antenna (3) for recording, treatment and analysis; said torque wrench further comprising a multicolored LED, a display panel or a sound transducer, said multicolored LED, display panel or sound transducer being configured to present information on the torque values read and connection status with the external console; said external console being a device among: computer, smartphone and/or tablet.

2. Electronic torque wrench, according to claim 1, **characterized by** being made of titanium and its alloys or other medical grade materials.

3. Electronic torque wrench, according to any one of claims 1 to 2, **characterized in that** the lower portion comprises a battery compartment (5) and a cover (6) configured to be screwed or fit by interference thereon.

4. Electronic torque wrench, according to any one of claims 1 to 3, **characterized by** being configured to apply torque to an implant or prosthesis screw in dental, cranio-maxillofacial and orthopedic surgeries, in addition to prosthetic procedures.

5. Electronic torque wrench, according to any one of claims 1 to 4, **characterized in that** it further comprises a sound transducer configured to provide information on connection status with the external console or torque values read.

6. System particularly used in dental and/or medical procedures **characterized by** comprising:
- an electronic torque wrench as defined in any one of claims 1 to 5;
- the external console, which comprises an embedded software;
the torque wench being configured to send all the torque values obtained through its use to the external console; and the external console being configured to record, treat and analyse said torque values.

## Patentansprüche

1. Elektronischer Drehmomentschlüssel, der zur Verwendung in zahnmedizinischen und/oder medizinischen Vorrichtungen konfiguriert ist, wobei der elektronische Drehmomentschlüssel umfasst:
- einen Abschnitt, der einen Aufsteckkopf (1) und einen Ratschenmechanismus (2) umfasst;
- einen unteren Abschnitt;
- einen mittleren Abschnitt, der Sensoren (9), eine HF-Antenne (3), einen HF-Sender (8), eine Leiterplatte (4) und eine Mikroprozessorschaltung (7) umfasst, wobei alle diese elektronischen Komponenten gegenüber Temperaturen beständig sind, die mit Sterilisationsverfahren wie Autoklavenprozessen in Zusammenhang stehen, und in dem mittleren Abschnitt versiegelt sind, der ferner ein geschweißtes Fenster umfasst, das den HF-Sender (8) und die Antenne (3) bedeckt, wobei das Fenster aus einem Material besteht, das HF-Strahlung durchlässt; wobei der obere, der mittlere und der untere Abschnitt nacheinander verbunden und in einen metallischen oder polymeren Körper eingewickelt sind, wobei der elektronische Drehmomentschlüssel elektronische Komponenten umfasst, die konfiguriert sind, um Temperaturen und Drücken ausgesetzt zu werden, die mit Sterilisationsverfahren, wie Autoklavenprozessen in Zusammenhang stehen, ohne den mitteren Abschnitt für derartige Verfahren zu zerlegen, wobei die Sensoren (9) so konfiguriert sind, dass sie einen auf den oberen Abschnitt ausgeübten Drehmomentwert erfassen und ein entsprechendes Signal erzeugen, wobei die Mikroprozessorschaltung (7) so konfiguriert ist, dass sie das Signal verschlüsselt und dem HF-Sender (8) zuführt, und wobei der HF-Sender (8) so konfiguriert ist, dass er das verschlüsselte Signal über die Antenne (3) zur Aufzeichnung, Behandlung und Analyse an eine externe Konsole sendet;
wobei der Drehmomentschlüssel ferner eine mehrfarbige LED, ein Anzeigefeld oder einen Schallwandler umfass, wobei die mehrfarbige LED, das Anzeigefeld oder der Schallwandler so konfiguriert sind, dass sie Informationen über die gelesenen Drehmomentwerte und den Verbindungsstatus mit der externen Konsole darstellen;
wobei es sich bei der externen Konsole um eine Vorrichtung aus der Gruppe Computer, Smartphones und/oder Tablets handelt.

2. Elektronischer Drehmomentschlüssel nach Anspruch 1, **dadurch gekennzeichnet, dass** er aus Titan und dessen Legierungen oder anderen medizintauglichen Materialien hergestellt ist.

3. Elektronischer Drehmomentschlüssel nach einem der Ansprüche 1 bis 2, **dadurch gekennzeichnet, dass** der untere Abschnitt ein Batteriefach (5) und eine Abdeckung (6) umfasst, die konfiguriert ist, um daran angeschraubt oder durch Einpressen angebracht zu werden.

4. Elektronischer Drehmomentschlüssel nach einem der Ansprüche 1 bis 3, **dadurch gekennzeichnet, dass** er so konfiguriert ist, dass er ein Drehmoment auf ein Implantat oder eine Prothesenschraube bei zahnärztlichen, kraniomaxillofazialen und orthopädischen Eingriffen zusätzlich zu prothetischen Prozeduren ausübt.

5. Elektronischer Drehmomentschlüssel nach einem der Ansprüche 1 bis 4, **dadurch gekennzeichnet, dass** er ferner einen Schallwandler umfasst, der so konfiguriert ist, dass er Informationen über den Verbindungsstatus mit der externen Konsole oder die gelesenen Drehmomentwerte bereitstellt.

6. System, das insbesondere bei zahnärztlichen und/oder medizinischen Prozeduren verwendet wird, **dadurch gekennzeichnet, dass** es umfasst:
- einen elektronischen Drehmomentschlüssel nach einem der Ansprüche 1 bis 5;
- die externe Konsole, die eine eingebettete Software umfasst;
wobei der Drehmomentschlüssel so konfiguriert ist, dass er alle durch seine Verwendung erhaltenen Drehmomentwerte an die externe Konsole sendet; und die externe Konsole so konfiguriert ist, dass sie die Drehmomentwerte aufzeichnet, verarbeitet und analysiert.

## Revendications

1. Clé dynamométrique électronique conçue pour être utilisée dans des appareils dentaires et/ou médicaux, ladite clé dynamométrique électronique comprenant :
- une partie supérieure comprenant une douille de tête (1) et un mécanisme à cliquet (2) ;
- une partie inférieure ;
- une partie centrale comprenant des capteurs (9), une antenne RF (3), un émetteur RF (8), une carte de circuit (4), un circuit de microprocesseur (7), tous ces composants électroniques étant résistants aux températures associées aux procédures de stérilisation telles que les processus d'autoclave et scellés dans ladite partie centrale qui comprend en outre une fenêtre soudée recouvrant l'émetteur RF (8) et l'antenne (3), la fenêtre est constituée d'un matériau qui autorise le passage du rayonnement RF ; dans laquelle les parties supérieure, centrale et inférieure sont reliées de manière séquentielle et enveloppées dans un corps métallique ou polymère, dans laquelle ladite clé dynamométrique électronique comprend des composants électroniques conçus pour être soumis aux températures et aux pressions associées aux procédures de stérilisation telles que les processus d'autoclave sans démonter la partie centrale pour de telles procédures, dans laquelle lesdits capteurs (9) sont conçus pour détecter une valeur de couple appliquée à la partie supérieure et pour générer un signal correspondant, ledit circuit de microprocesseur (7) est conçu pour crypter et transmettre ledit signal à l'émetteur RF (8), et ledit émetteur RF (8) est conçu pour envoyer ledit signal crypté à une console externe par l'intermédiaire de l'antenne (3) en vue de son enregistrement, son traitement et son analyse ;
ladite clé dynamométrique comprenant en outre une DEL multicolore, un panneau d'affichage ou un transducteur sonore, ladite DEL multicolore, ledit panneau d'affichage ou ledit transducteur sonore étant conçu(e) pour présenter des informations sur les valeurs de couple lues et l'état de liaison avec la console externe ;
ladite console externe étant un dispositif parmi : un ordinateur, un téléphone intelligent et/ou une tablette.

2. Clé dynamométrique électronique, selon la revendication 1, **caractérisée en ce qu'**elle est constitué de titane et ses alliages ou d'autres matériaux de qualité médicale.

3. Clé dynamométrique électronique, selon l'une quelconque des revendications 1 à 2, **caractérisée en ce que** la partie inférieure comprend un compartiment à batterie (5) et un couvercle (6) conçu pour être vissé ou fixé par interférence sur celui-ci.

4. Clé dynamométrique électronique, selon l'une quelconque des revendications 1 à 3, **caractérisée en ce qu'**elle est conçue pour appliquer un couple à une vis d'implant ou de prothèse lors de procédures dentaires, cranio-maxillo-faciales et orthopédiques, en plus des procédures prothétiques.

5. Clé dynamométrique électronique, selon l'une quelconque des revendications 1 à 4, **caractérisée en ce qu'**elle comprend en outre un transducteur sonore conçu pour fournir des informations sur l'état de liaison avec la console externe ou des valeurs de couple lues.

6. Système particulièrement utilisé dans les procédures dentaires et/ou médicales **caractérisé en ce qu'**il comprend :
- une clé dynamométrique électronique selon l'une quelconque des revendications 1 à 5 ;
- la console externe, qui comprend un logiciel intégré ;
la clé dynamométrique étant conçue pour envoyer toutes les valeurs de couple obtenues par l'intermédiaire de son utilisation à la console externe ; et la console externe étant conçue pour enregistrer, traiter et analyser lesdites valeurs de couple.
